(19) Europäisches Patentamt
European Patent Office
Office européen des brevets

(11) EP 0 872 556 A2

(12) # EUROPÄISCHE PATENTANMELDUNG

(43) Veröffentlichungstag:
**21.10.1998 Patentblatt 1998/43**

(51) Int. Cl.[6]: **C12P 19/26**, C12P 19/44

(21) Anmeldenummer: **98106423.1**

(22) Anmeldetag: **08.04.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.04.1997 DE 19716013**

(71) Anmelder:
**HOECHST AKTIENGESELLSCHAFT**
**65929 Frankfurt am Main (DE)**

(72) Erfinder:
- **Vértesy, Laszlò, Dr.**
  **65817 Eppstein (DE)**
- **Stärk, Andreas, Dr.**
  **65817 Eppstein (DE)**
- **Ehlers, Eberhard, Dr.**
  **65719 Hofheim (DE)**

(54) **Verfahren zur Herstellung von Moenomycin A**

(57) Moenomycin A läßt sich herstelle durch Fermentation eines Moenomycin produzierenden Mikroorganismus und nachfolgende Abtrennung des Moenomycin A von den übrigen Komponenten des Kulturfiltrats durch Chromatographie, wobei als Chromatographiematerial ein Anionenaustauscher benutzt wird.

EP 0 872 556 A2

**Beschreibung**

Die vorliegende Erfindung betrifft ein Verfahren zur Gewinnung von Moenomycin A aus mikrobiellen Kulturflüssigkeiten.

Das Moenomycin sowie viele seiner Derivate sind schon seit langem bekannt (vgl. DE-OS 3 704 659, EP 0 355 679, G. Huber in "Antibiotics", ed. F. Hahn, Springer Verlag, Berlin 1979, Vol. IV, Seite 135 ff., Welzel et al. in Tetrahedron, 1983, Vol. 39, No. 9. 1583-1591). Moenomycin, z.B. das Moenomycin A, werden vorzugsweise durch Fermentation von Mikroorganismen und nachfolgende Aufreinigung gewonnen.

Unter dem Begriff Moenomycin im Sinne der vorliegenden Patentanmeldung ist ein Komplex von Moenomycin Komponenten (z.B. wie er von Mikroorganismen gebildet wird) sowie auch die einzelnen Komponenten zu verstehen. Das Moenomycin A ist eine antibiotisch besonders wirksame Komponente des Moenomycin-Komplexes.

Das genannte Moenomycin A hat die folgende Strukturformel:

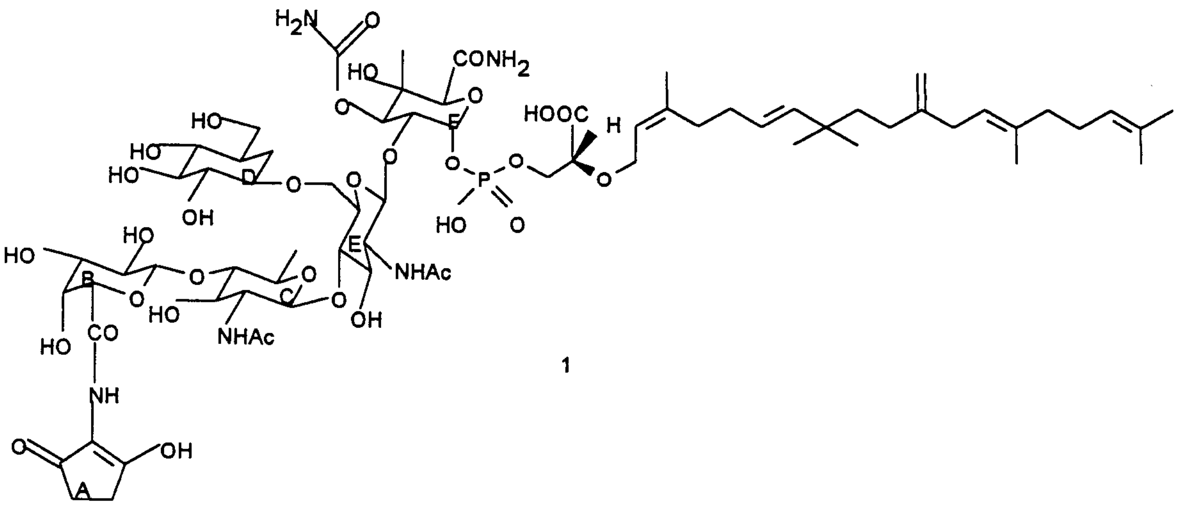

1

Die wichtigsten Komponenten neben Moenomycin A sind die Komponenten $A_{11}$, $A_{12}$, $C_1$, $C_2$, $C_3$ und $C_4$.

Mikroorganismen, die Moenomycin-Komplexe produzieren sind z.B. Streptomyces bambergiensis, -ghanaensis, -ederensis und -geysirensis. Besonders bevorzugt ist Streptomyces bambergiensis (vgl. in diesem Zusammenhang Huber a.a.O.).

Es ist in neuerer Zeit gefunden worden, daß Moenomycin, insbesondere aber Moenomycin A, geeignet ist zur Kontrolle von Helicobacter pylori-Infektionen (HOE 93/F 392). Die Infektionen mit Helicobacter pylori sind die Hauptursache von Gastritis bzw. Ulcera des Menschen.

Für den Einsatz in der Humanmedizin ist der Moenomycin-Komplex zwar geeignet, aber wegen der wechselnden Komponentenzusammensetzung, die eine Folge der fermentationsabhängigen mikrobiologischen Genese ist, ist die Dosierung problembehaftet. Deshalb geht man in der Humanmedizin dazu über, nur einheitliche Verbindungen (Monokomponenten) als Wirkstoffe einzusetzen.

Nach "Antimicrobial Agents and Chemotherapy", 1965, 737, lassen sich aus dem Moenomycin-Komplex durch Chromatographie an Kieselgelsäulen und Elution mit Propanol-Ammoniak-Gemischen nach Fraktionierung vier Komponenten A, $B_1$, $B_2$ und C isolieren, die auch auf dem Dünnschichtchromatogramm nach Entwickeln des Chromatogramms im System n-Propanol/2 n-$NH_3$ (70:30) und Lokalisierung durch Einsprühen mit Chlorsulfonsäure/Eisessig als diskrete Flecken mit unterschiedlichen $R_F$-Werten nachweisbar sind. Die Moenomycin-Komponenten sind einander chemisch sehr ähnlich und zeigen hinsichtlich Löslichkeit, Säureeigenschaft, Farbreaktionen und Verhalten bei der Papierchromatographie und Papierelektrophorese keine Unterschiede zum Moenomycin-Komplex; sie unterscheiden sich voneinander durch ihr chromatographisches Verhalten an Kieselgel sowie durch das Fehlen einer charakteristischen UV-Absorption bei den Komponenten $B_1$ und $B_2$.

In neuerer Zeit wurde zur Komponententrennung die Umkehrphasen-(RP)-Chromatographie z.B. an LiChroprep®RP-18 herangezogen (J. Scherkenbeck et al. Tetrahedron, Vol. 49, No. 15, 3091 - 3100, 1993).

Keine dieser Labormethoden ist jedoch für die technische Gewinnung der Moenomycin-Komponenten geeignet: die Kieselgel-Chromatographie ist wegen der schlechten Wiederverwendbarkeit des Trägers und wegen des Preises nicht zeitgemäß, die Umkehrphasen-Chromatographie ist mit sehr hohen Kosten behaftet. Der Einsatz von anderen Trennprinzipien wie z.B. von Ionenaustauschern scheiterte bislang an der ungenügenden Abtrennung der Moenomycin-A-Komponente von den C-Komponenten. In den Patentschriften GB 1068639 und DE 1236726 ist eine Reinigung des Moenomycin-Komplexes an Ionenaustauscher auf Cellulosebasis beschrieben. Diese Reinigung ist jedoch nicht geeignet zur Komponententrennung. Darüber hinaus ist dieses Verfahren vor allem wegen der Druckempfindlichkeit des Celluloseträgers nur schwer in den technischen Maßstab übertragbar. Die DE-Patentschrift 1 617 466 berichtet über Trennungen der Moenomycine D, E, F, G und H an stark basischen Anionenaustauschern auf Polystyrolbasis. Zur Reinigung der Moenomycin-A-Komponente ist jedoch das beschriebene Verfahren, mangels Selektivität nicht geeignet: Die Trennung der A-Komponente von den C-Komponenten ist unzureichend.

Es ist nun überraschenderweise gefunden worden, daß das Moenomycin A sich aus dem Moenomycin-Komplex kostengünstig in großem Maßstab mittels Anionenaustauscherchromatographie abtrennen läßt.

Gegenstand der vorliegenden Erfindung ist demzufolge ein Verfahren zur Herstellung von Moenomycin A durch Fermentation eines Moenomycinproduzierenden Mikroorganismus und nachfolgende Abtrennung des Moenomycin A von den übrigen Komponenten des Kulturfiltrats durch Chromatographie, dadurch gekennzeichnet, daß als Chromatographiematerial ein Anionenaustauscher benutzt wird.

Bevorzugte Anionenaustauschermaterialien sind modifizierte Methacrylat-Copolymere mit Diethylaminoethyl-Gruppen als funktionelle Gruppen.

Besonders geeignete Anionenaustauschermaterialien sind die Produkte ®Toyopearl DEAE-650 S (TOSOHAAS, Montgomeryville, PA 18936 USA) oder ®Fractogel TSK DEAE-650 S (E. Merck, Darmstadt).

Die erfindungsgemäße Chromatographie kann in dem pH-Bereich von 5 bis 9, vorzugsweise zwischen pH 7 und 8 stattfinden. Es eignen sich viele Puffer-Systeme zur Trennung, die im genannten pH-Bereich wirksam sind, wie z.B. Phosphat-, Citrat, Tris/HCl-Puffer oder andere.

Zur Elution (Desorption) der Moenomycin-Komponenten vom Anionenaustauscher können ansteigende Salzgradienten, vorzugsweise 0 bis 4, insbesondere 0 bis 2 molare Gradienten angewandt werden. Besonders geeignete Salze sind NaCl und KCl.

Es kann weiterhin sinnvoll sein, abfallende pH-Gradienten zu benutzen, vorzugsweise von pH 9 bis pH 3, insbesondere von pH 8 bis pH 4,5. Solch einen pH-Gradienten kann man erzeugen, indem man z.B. von 1/15 m $Na_2HPO_4$ (~pH 8) ausgehend linear zu 1/15 m $KH_2PO_4$ (~pH 4,9)-Puffer in an sich bekannter Weise wechselt.

Die Trennung am Anionenaustauscher kann gegebenenfalls auch mit Puffersystemen durchgeführt werden, die einen organischen Lösungsmittelanteil enthalten. Verwendbar sind z.B. aliphatische Alkohole wie z.B. Methanol, Ethanol, die Propanole oder andere in Konzentrationen von 0 bis 95 %, vorzugsweise 15 bis 40 %. Die Kapazität des Ionenaustauschers nimmt ab mit zunehmender organischer Lösungsmittelkonzentration, somit ist auch das Eluieren der Moenomycin-Komponenten mit einem prozentual ansteigenden Lösungsmittelgradienten möglich. Von den Moenomycin-Komponenten wird das Moenomycin A vom erfindungsgemäßen Träger am schwächsten gebunden, mit der Folge, daß bei der Elution diese Verbindung zuerst gewonnen wird.

Für die erfindungsgemäße Abtrennung des Moenomycin A mittels Anionenaustauscher ist eine Vorreinigung des Kulturfiltrats vorteilhaft. Diese Vorreinigung kann durch Anwendung eines neutralen Adsorptionsharzes erfolgen. Geeignete neutrale Adsorptionsharze sind z.B. MCI GEL CHP20P® bzw. DIAION® HP 20SS (Mitsubishi Chemical Corporation) oder Amberlite® XAD 16 bzw. XAD 1180S (Rohm & Haas). Man bringt hierbei das Kulturfiltrat mit dem Adsorptionsharz in Berührung, trennt das beladene Harz vom ausextrahierten Kulturfiltrat ab und eluiert (desorbiert) die Antibiotika mit Mischungen von organischen Lösungsmitteln in Wasser in an sich bekannter Weise. So eine Vorreinigung an neutralen Adsorptionsharzen bewirkt eine Entsalzung, Entfettung und Konzentrierung des rohen Moenomycins und begünstigt damit die Trennschärfe des Ionenaustauscher-Schrittes.

Eine andere Methode der Vorreinigung besteht darin, daß das Kulturfiltrat, gegebenenfalls in eingeengter oder getrockneter Form mit einem unpolaren Lösungsmittel extrahiert wird, wobei unpolare Verunreinigungen sich in dem unpolaren Lösungsmittel lösen. Geeignete unpolare Lösungsmittel sind z.B. Petrolether, Aceton, Methylethylketon und andere. Aus dem verbleibenden Kulturfiltrat läßt sich anschließend der Moenomycin-Komplex mit einem polaren Lösungsmittel, wie z.B. Methanol, extrahieren. Das gegebenenfalls sinnvolle Einengen oder Trocknen des Kulturfiltrats erfolgt z.B. durch Destillation, Ultrafiltration, Sprühtrocknen oder Gefriertrocknen.

Um die Extraktion mit dem polaren Lösungsmittel effektiver zu gestalten, kann es sinnvoll sein, dem Kulturfiltrat einen Komplexbildner wie EDTA, Citronensäure oder ähnliches zuzusetzen. Der Komplexbildner reduziert die Konzentration an mehrwertigen Ionen, die mit Moenomycin Salze bilden, die in dem polaren Lösungsmittel schlecht löslich sind.

Vorteilhafte Vorgehensweisen zur Anreicherung des Moenomycin bzw. des Moenomycins A sind in den Beispielen 1 und 2 beschrieben.

Nach der Vorreinigung und der Komponenten-Trennung an modifizierten Methacrylat-Copolymer-DEAE Trägern erhält man salzhaltige Moenomycin-A-Lösungen, die mit geringen Mengen anderer Komponenten, insbesondere Moenomycin $A_{12}$ verunreinigt sein können. Die Reinheit des Moenomycins A beträgt 90 bis über 99 % je nach Zusammensetzung des Säuleneluates der Komponententrennung. Für die Endreinigung , d.h. Abreicherung der restlichen Nebenkomponenten und Entsalzung verwendet man vorteilhafterweise erneut neutrale Adsorptionsharze wie vorzugsweise MCI Gel CHP20P®, bzw. DIAION®HP20SS oder Amberlite®XAD 16 bzw. XAD 1180 S. Auftrennungen an solchen Harzen mit wäßrigen Puffer/organische Lösungsmittel-Mischungen, wie z.B. Phosphatpuffer/Isopropanol-Mischungen können erhebliche Reinigungen des Moenomycins A bewirken. So kann z.B. nur unvollständig angereichertes Moenomycin A bis zu über 99 %iger Reinheit raffiniert werden. Geeignet sind viele Puffersubstanzen, die zwischen pH 4 und 10 ausreichend puffern, vorzugsweise zwischen pH 7 und 9.

Als organische Lösungsmittel kommen mit Wasser mischbare Lösemittel, wie niedere Alkohole, Aceton, Acetonitril u.ä. in Frage.

Die Entsalzung der mit Salzen belasteten Moenomycin A-Lösungen kann durch Verwendung der genannten neutralen Adsorptionsharze, geschehen. Nach der Beladung bei pH-Werten zwischen 7 und 9 wird die Elution mit Wasser vorgenommen, dem steigende Anteile eines mit Wasser mischbaren organischen Lösungsmittels hinzugefügt worden sind. Das Moenomycin A enthaltende Säuleneluat mit genügender Komponenten-Reinheit wird gesammelt, durch Ultrafiltration oder Destillation, vorzugsweise im Vakuum, konzentriert und getrocknet. So erhaltenes Moenomycin A hat eine Reinheit von über 97 %. Als Verunreinigung liegt hauptsächlich die Moenomycin-Komponente $A_{12}$ vor.

Will man höherprozentiges Moenomycin A herstellen, so können die Reinigungsschritte Ionenaustauscher-Chromatographie und Komponentenreinigung an Adsorptionsharzen wiederholt werden. Man erhält ≥ 99 %iges Moenomycin. Je nach Wahl der Salze und Puffer bei den Trennungen fällt das Antibiotikum als Natrium-, Kalium-, Ammonium-Salz oder als sonstiges Salz an. Welchem Gegenion (Kation) der Vorzug gegeben wird, kann von den galenischen Erfordernissen abhängend frei gewählt werden.

Durch die folgenden Beispiele und durch den Inhalt der Patentansprüche soll die vorliegende Erfindung näher erläutert werden.

Beispiel 1

Anreicherung des Moenomycins aus dem Kulturfiltrat durch Festphasen-Extraktion

Durch Fermentation einer Mutante des Streptomyces ghanaensis (ATCC 14672), die in Anlehnung an die Vorschrift von K.H. Wallhäuser et al. Antimicrobial Agents and Chemotherapy, 1965, Seite 734 - 736 durchgeführt worden ist, sowie durch Filtration mittels einer Simex Filterpresse, Nachwaschen mit entsalztem Wasser und Klarfiltration über KS 80 Mehrschichtenfilter werden 1.100 l Moenomycin-haltiges Kulturfiltrat gewonnen. Feststoffgehalt ~20g/l mit 2,8 g Moenomycin A. Leitfähigkeit: 14 mS/cm, pH 7,5.

150 l mit 3 kg Feststoff und 420 g Moenomycin A werden auf eine 58 l fassende (BxH = 33,3 cm x 66 cm), mit Adsorptionsharz MCI® Gel CHP20P gefüllte Säule aufgetragen. Flußrate: 2,5 l pro Minute. Nach der Adsorption wird mit einem 0 bis 40 % Isopropanol in Wasser enthaltenden Gradienten eluiert mit einem Durchfluß von 10 l pro Minute. Die überwiegend Moenomycin A-haltigen Fraktionen werden zusammengefaßt, durch Ultrafiltration konzentriert und getrocknet. Es resultieren 0,7 kg des Moenomycin-Komplexes.

Beispiel 2

Anreicherung des Moenomycins aus dem Kulturfiltrat durch selektives Lösen.

1.000 Liter Kulturfiltrat, hergestellt nach Beispiel 1, werden mit einer "Industriellen Pilotanlage UF-36-9" (DDS) durch Ultrafiltration auf 140 l konzentriert. Als Membranen werden 9 $m^2$ ®Nadir PA 20 H-Schichten verwendet. Die Ultrafiltrationsgeschwindigkeit (Durchfluß) beträgt 28 Liter pro $m^2$ und Stunde. Bei Erreichen eines Retenatvolumens von 140 l wird das Konzentrat kontinuierlich mit insgesamt 140 l entsalztem Wasser gewaschen. Hierbei fällt die Leitfähigkeit des Konzentrates auf 1.8 mS/cm. Die Endkonzentration des Moenomycin-Komplexes im Retenat (140 l) beträgt 32,9 g pro Liter, Der pH-Wert 6,9. Das Retenat wird sprühgetrocknet. Das Ultrafiltrat (Permeat) beinhaltet 24 mg Moenomycin pro Liter, insgesamt 24 g, entsprechend 0,52 % der Ausgangsmenge. Das Endprodukt (das getrocknete Retenat) ist ein hellbraunes Pulver, wiegt 15 kg und enthält 2,8 kg Moenomycin A (99 % der eingesetzten A-Komponente).

3 kg des Sprühtrocknungsproduktes werden mit 50 l Aceton aufgerührt und abzentrifugiert. Der organische Extrakt beinhaltet 1,02 kg Lipide. Der ungelöste Rückstand wird 3mal mit je 20 l Methanol 1 Stunde ausgerührt und anschließend abfiltriert. In den Methanolphasen befindet sich Moenomycin A (0,54 kg). Die Leitfähigkeit einer 1 %igen Lösung der von Methanol befreiten Substanz (1,1 kg):1 mS/cm. Der methanolische Extrakt kann in Vakuum eingeengt und mit Wasser verdünnt zur Komponententrennung unmittelbar eingesetzt werden.

Beispiel 3

Trennung der Moenomycin-Komponenten A von C durch Ionenaustauscher-Chromatographie

1 kg Moenomycin-Komplex wird in 20 l Wasser gelöst (pH 7,4) und auf eine, mit 20 l bei pH 7,4 äquilibrierten Anionenaustauscher, Fractogel® TSK DEAE-650, gefüllten Säule aufgetragen. Man wäscht zunächst mit Puffer A (20 mM Phosphatpuffer, pH 7,4. 2,5 mS + 20 % Propanol-2) und legt dann einen linearen Gradienten von Puffer A nach Puffer B (= 1 M NaCl in Puffer A) an. Die Flußrate beträgt hierbei 7 l/Minute, entsprechend 21 Säulenvolumina in der Stunde, die Fraktionsgröße 20 l (~1 Säulenvolumen). Nach Entsalzung der überwiegend Moenomycin A-haltigen Fraktionen durch Ultrafiltration und Trocknung ergibt die Trennung 480 g Komponente A in 95 %iger Reinheit. Die Wiedergewinnungsrate der Moenomycinkomponenten ist größer als 90 %.

Beispiel 4

Entsalzung des am Anionenaustauscher getrennten Moenomycin A an Adsorptionsharzen

81 g 95 %iges Moenomycin A, gelöst in 7 l wäßriger Pufferlösung, pH 7,9, 10,5 mS/cm, wie es nach der DEAE-Reinigung und Ultrafiltration anfällt, werden auf eine 2,7 l fassende (BxH = 10cm x 35cm), mit MCI-Gel® CHP20P gefüllte Säule aufgetragen. Eluiert wird mit einem 0 - 35 % Isopropanol in Wasser enthaltendem Gradienten. Die Fraktionen die 97 % Moenomycin A enthalten, werden zusammengefaßt, durch Ultrafiltration eingeengt und gefriergetrocknet. 55 g 98,5 %iges Moenomycin A neben 24 g 88 %igem Material, werden erhalten.

Beispiel 5

Nachreinigung des Moenomycin A am Adsorptionsharz MCI-Gel CHP20P

81 g 95 %iges Moenomycin A, gelöst in 7 l wäßriger Pufferlösung, pH 7,9, Leitfähigkeit 10,5 mS/cm, wie es nach der DEAE-Reinigung und Ultrafiltration anfällt, werden auf eine 2,7 l fassende (B x H = 10 cm x 35 cm), mit MCI Gel® CHP20P gefüllte Säule aufgetragen. Eluiert wird mit einem linearen Gradienten von Puffer A (m/15 Phosphatpuffer, pH 7,8) nach Puffer B (m/15 Phosphatpuffer, pH 7,8 mit 33 % Propanol-2). Es wird der Säulenfluß fraktioniert und analysiert. Die Fraktionen mit mindestens 98,8 %igem Moenomycin A werden gesammelt, wie in Beispiel 4 beschrieben entsalzt und getrocknet: 71 g 99,3 %iges Moenomycin A, mit 0,4 % Moenomycin $A_{12}$ als Begleitstoff.

**Patentansprüche**

1. Verfahren zur Herstellung von Moenomycin A durch Fermentation eines Moenomycin produzierenden Mikroorganismus und nachfolgende Abtrennung des Moenomycin A von den übrigen Komponenten des Kulturfiltrats durch Chromatographie, dadurch gekennzeichnet, daß als Chromatographiematerial ein Anionenaustauscher benutzt wird.

2. Verfahren gemäß Anspruch 1, dadurch gekennzeichnet, daß das Anionenaustauschermaterial ein modifiziertes Methacrylat-Copolymer mit Diethylaminoethyl-Gruppen als funktionelle Gruppen ist.

3. Verfahren gemäß den Ansprüchen 1 oder 2, dadurch gekennzeichnet, daß als Elutionsmittel eine Salzlösung mit einem ansteigenden Salzgehalt von 0 bis 4 molar benutzt wird.

4. Verfahren gemäß einem oder mehreren der Ansprüche 1 bis 3, dadurch gekennzeichnet, daß als Elutionsmittel ein Lösungsmittel mit einem fallenden pH zwischen pH 9 bis pH 3 benutzt wird.

5. Verfahren gemäß einem oder mehreren der Ansprüche 1 - 4, dadurch gekennzeichnet, daß das Kulturfiltrat vor der Anionenaustauscher-Chromatographie einer Vorreinigung durch Chromatographie an einem neutralen Adsorptionsharz unterzogen wird.

6. Verfahren gemäß einem oder mehreren der Ansprüche 1 - 5, dadurch gekennzeichnet, daß das Kulturfiltrat vor der Anionenaustauscher-Chromatographie einer Vorreinigung durch Extraktion mit einem unpolaren Lösungsmittel unterzogen wird.

7. Verfahren gemäß einem oder mehreren der Ansprüche 1 - 6, dadurch gekennzeichnet, daß das erhaltene Moenomycin A durch mindestens einen weiteren Chromatographieschritt an einem neutralen Adsorptionsharz weiter gereinigt wird.